# EUROPEAN PATENT APPLICATION

(11) **EP 1 043 298 A1**
(43) Date of publication of application: **11.10.2000**
(21) Application number: 99943377.4
(22) Date of filing: 16.09.1999
(51) Int. Cl.: C07C 29/40, C07C 29/36, C07C 31/137

(54) **PROCESSES FOR THE PREPARATION OF ALCOHOLS**

(30) Priority: 18.09.1998 JP 28332098; 18.09.1998 JP 28338698
(71) Applicant: Daicel Chemical Industries, Ltd., Osaka 590-8501 (JP)
(72) Inventor: SHIMOJITOSYO, Hiroshi, Osaka-shi, Osaka 555-0023 (JP); NAKANO, Tatsuya, Himeji-shi, Hyogo 670-0094 (JP)
(74) Representative: Grünecker, Kinkeldey, Stockmair & Schwanhäusser Anwaltssozietät
(86) International application number: JP9905062
(87) International publication number: WO0017139

(57) **Abstract**

A carbonyl compound having the following formula (1): 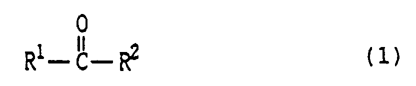 wherein R¹ is a non-aromatic hydrocarbon group having a tertiary carbon atom at a bonding site to a carbonyl group, and R² is a hydrogen atom or a hydrocarbon group, is reacted with an organic titanium compound having the following formula (2):

R³ₙTiL₄₋ₙ (2)

wherein R³ is a hydrocarbon group, L is a ligand, and n denotes an integer of 1 to 4, to yield an alcohol having the following formula (3): 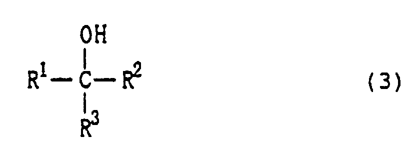 wherein R¹, R² and R³ have the same meanings as defined above.

According to another process of the invention, a 1-adamantanecarboxylic acid derivative having the following formula (4): 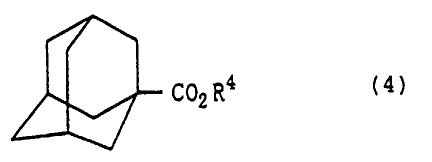 wherein R⁴ is a hydrogen atom or a hydrocarbon group, and an adamantane ring may have a substituent, is reacted with an organic magnesium compound having the following formula (5):

R⁵MgX (5)

wherein R⁵ is a C₁-C₆ alkyl group, and X is a halogen atom, to yield an adamantanemethanol derivative having the following formula (6): 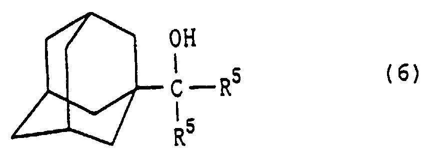 wherein R⁵ has the same meaning as defined above, and the adamantane ring may have a substituent.

## Description

### Technical Field

The present invention relates to processes for producing alcohols. Specifically, the present invention relates to processes for producing secondary or tertiary alcohols each having a quaternary carbon atom at an adjacent position to a carbon atom bonded to a hydroxyl group, and relates to processes for producing adamantanemethanol derivatives which are useful as materials for photosensitive resins and other functional polymers and for pharmaceuticals.

### Background Art

Processes utilizing an addition reaction of a carbonyl compound with an organic metallic compound are known as processes for producing alcohols. For example, a reaction of an aldehyde or a ketone with a Grignard reagent produces a corresponding secondary or tertiary alcohol. However, when a carbonyl compound having a quaternary carbon atom at the adjacent position to a carbonyl group is used as a reactant in this technique, the yield of a target alcohol is markedly low, as the addition reaction proceeds slowly or a reduction reaction or a coupling reaction of the carbonyl compound molecules with each other induced by the formation of an enolate precedes the addition reaction.

J. Am. Chem. Soc., 1989, vol. 111, pp. 4392 proposes a process of reacting a carbonyl compound with a Grignard reagent in the presence of cerium chloride to yield a corresponding alcohol. According to this process, the addition reaction of the Grignard reagent relatively smoothly proceeds, even when the reactant is a carbonyl compound having a quaternary carbon atom at the adjacent position to a carbonyl group. However, when the reactant carbonyl compound has a rigid and bulky hydrocarbon group bonded to the carbonyl group, the reaction yield significantly decreases even according to this process.

Separately, α,α-dimethyl-1-adamantanemethanol and other adamantanemethanol derivatives are expected to be material compounds for monomer constituents of photoresist resins or material compounds for antibiotics and other pharmaceuticals (U.S. Pat. No. 3284445).

U.S. Pat. No. 3284445 discloses a process for producing α,α-dimethyl-1-adamantanemethanol by reacting 1-adamantanoyl chloride with methylmagnesium bromide. However, the reactant 1-adamantanoyl chloride used in this process is unstable and must be handled with care. The reactant 1-adamantanoyl chloride is obtained by allowing thionyl chloride, phosphorus pentachloride, or another halogenating agent to act on 1-adamantanecarboxylic acid. These halogenating agents are unstable and must be handled with care. In addition, a highly toxic gas such as sulfur dioxide gas is evolved in the reaction or large amounts of by-products containing sulfur or phosphorus are formed and exhausted in an after-treatment. The process in question is not desirable also from the viewpoint of environmental protection.

### Disclosure of Invention

Accordingly, an object of the invention is to provide a process for producing an alcohol through an addition reaction of a carbonyl compound having a quaternary carbon atom at the adjacent position to a carbonyl group, which process can be applied for general purpose use.

Another object of the invention is to provide a process for producing an alcohol, in which an addition reaction smoothly proceeds to yield a corresponding alcohol in a high yield even when the reactant is a carbonyl compound having a rigid and bulky group such as an adamantyl group.

A further object of the invention is to provide a process for producing an adamantanemethanol derivative in a high yield from a material that is easily available without by-producing environmentally undesirable substances and can be easily handled.

The present inventors made intensive investigations to achieve the above objects and found that the use of a specific organic titanium compound can smoothly proceed an addition reaction to yield a corresponding alcohol in a high yield even when a carbonyl compound having a rigid and bulky group such as an adamantyl group is used, and that the action of an organic magnesium compound upon 1-adamantanecarboxylic acid or an ester thereof can yield a corresponding adamantanemethanol derivative in a high yield. The present invention has been accomplished based on these findings.

Specifically, the invention provides in an aspect a process for producing an alcohol (hereinafter briefly referred to as "alcohol production process 1"). The process includes the step of reacting a carbonyl compound having the following formula (1): 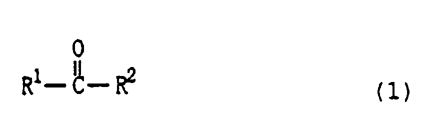 wherein R¹ is a non-aromatic hydrocarbon group having a tertiary carbon atom at a bonding site to a carbonyl group, and R² is a hydrogen atom or a hydrocarbon group,
with an organic titanium compound having the following formula (2):

R³ₙTiL₄₋ₙ (2)

wherein R³ is a hydrocarbon group, L is a ligand, and n denotes an integer of 1 to 4,
to yield an alcohol having the following formula (3): 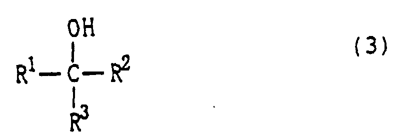 wherein R¹, R² and R³ have the same meanings as defined above.

In another aspect, the invention provides a process for producing an alcohol (hereinafter referred to as "alcohol production process 2"). The process includes the step of reacting a 1-adamantanecarboxylic acid derivative having the following formula (4): 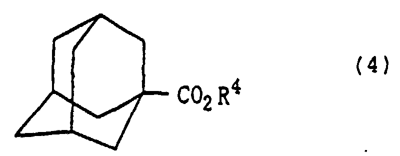 wherein R⁴ is a hydrogen atom or a hydrocarbon group, and an adamantane ring may have a substituent,
with an organic magnesium compound having the following formula (5):

R⁵MgX (5)

wherein R⁵ is a C₁-C₆ alkyl group, and X is a halogen atom, to yield an adamantanemethanol derivative having the following formula (6): 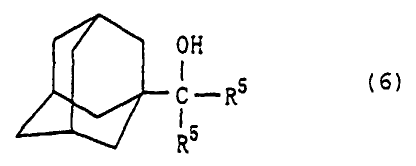 wherein R⁵ has the same meaning as defined above, and the adamantane ring may have a substituent.

### Best Mode for Carrying Out the Invention

In the formula (1) in the invented alcohol production process 1, the non-aromatic hydrocarbon group R¹ is not critical as far as the group is a non-aromatic hydrocarbon group having a tertiary carbon atom at a bonding site to a carbonyl group.

Such non-aromatic hydrocarbon groups include, but are not limited to, (a) aliphatic hydrocarbon groups each having a tertiary carbon atom at a bonding site to the carbonyl group, (b) alicyclic hydrocarbon groups each having a hydrocarbon group bonded to a carbon atom at a bonding site to the carbonyl group, or (c) bridged cyclic groups, each of which is bonded to the carbonyl group at a bridgehead position.

Illustrative examples of the aliphatic hydrocarbon groups (a) each having a tertiary carbon atom at a bonding site to the carbonyl group are t-butyl group, 1,1-dimethylpropyl group, 1-ethyl-1-methylpropyl group, 1,1-diethylpropyl group, 1,1-dimethylbutyl group, 1,1-dimethyl-2-propenyl group, and other aliphatic hydrocarbon groups (alkyl groups, alkenyl groups or alkynyl groups) having a tertiary carbon atom as a carbon atom at the bonding site and having about 4 to 10 carbon atoms and preferably about 4 to 6 carbon atoms.

The alicyclic hydrocarbon groups (b) each having a hydrocarbon group bonded to the carbon atom at the bonding site to the carbonyl group include, but are not limited to, 1-methylcyclopentyl group, 1-methylcyclohexyl group, 1-ethylcyclohexyl group, 1-isopropylcyclohexyl group, 1-propylcyclohexyl group, 1-methylcyclooctyl group, 1-methylcyclodecyl group, 1-methylcyclopentadecyl group, 1-methyl-2-cyclohexenyl group, and other alicyclic hydrocarbon groups (cycloalkyl groups or cycloalkenyl groups) having about 3 to 20 members and preferably about 5 to 15 members. These alicyclic hydrocarbon groups each have an aliphatic hydrocarbon group (e.g., an alkyl group) bonded to the carbon atom at the bonding site, which aliphatic hydrocarbon group has about 1 to 10 carbon atoms and preferably about 1 to 4 carbon atoms.

The bridged cyclic groups (c), each of which is bonded to the carbonyl group at a bridgehead position include, but are not limited to, bicyclo[1.1.0]but-1-yl group, bicyclo[2.2.0]hex-1-yl group, bicyclo[3.1.0]hex-1-yl group, bicyclo[2.2.1]hept-1-yl group, bicyclo[2.2.1]-2-hepten-1-yl group, bicyclo[3.1.1]hept-1-yl group, bicyclo[3.1.1]-2-hepten-1-yl group, bicyclo[4.1.0]hept-1-yl group, bicyclo[2.2.2]oct-1-yl group, bicyclo[3.2.1]oct-1-yl group, bicyclo[4.3.0]non-1-yl group, decalin-4a-yl group, bicyclo[3.3.3]undec-1-yl group, and other bridged bicyclic groups; tricyclo[2.2.1.0^{2,6}]hept-1-yl group, quadricyclan-1-yl group, adamant-1-yl group, tricyclo[4.3.1.1^{2,5}]undec-1-yl group, tricyclo[4.2.2.1^{2,5}]undec-1-yl group, tricyclo[4.2.1.1^{2,5}]dec-1-yl group, tricyclo[5.2.1.0^{2,6}]dec-1-yl group, and other bridged tricyclic groups.

The non-aromatic hydrocarbon groups may have a variety of substituents. Such substituents include, but are not limited to, halogen atoms, oxo group, hydroxyl group, mercapto group, substituted oxy groups (e.g., alkoxy groups, aryloxy groups, and acyloxy groups), substituted thio groups, carboxyl group, substituted oxycarbonyl groups, substituted or unsubstituted carbamoyl groups, cyano group, nitro group, substituted or unsubstituted amino groups, alkyl groups, alkenyl groups, alkynyl groups, cycloalkyl groups, cycloalkenyl groups, aryl groups (e.g., phenyl and naphthyl groups), aralkyl groups, and heterocyclic groups.

Hydrocarbon groups R² include, for example, methyl, ethyl, propyl, isopropyl, butyl, isobutyl, s-butyl, t-butyl, pentyl, hexyl, octyl, decyl, allyl, propargyl, and other aliphatic hydrocarbon groups (alkyl groups, alkenyl groups or alkynyl groups) each having about 1 to 20 carbon atoms; cyclopropyl, cyclobutyl, cyclopentyl, cyclohexyl, cyclooctyl, and other alicyclic hydrocarbon groups (cycloalkyl groups or cycloalkenyl groups) each having about 3 to 15 members; and phenyl, naphthyl, and other aromatic hydrocarbon groups. These hydrocarbon groups may have substituents similar to those mentioned above.

Preferred substituent R² includes hydrogen atom and C₁-C₁₀ aliphatic hydrocarbon groups, of which C₁-C₆ aliphatic hydrocarbon groups, particularly, methyl, ethyl, propyl, butyl, and other C₁-C₄ aliphatic hydrocarbon groups are typically preferred as R². Among them, C₁-C₄ alkyl groups are especially desirable.

Illustrative compounds of the formula (1) include, but are not limited to, t-butyl methyl ketone, t-butyl ethyl ketone, methyl 1,1-dimethylpropyl ketone, 1,1-diethylpropyl methyl ketone, and other dialkyl ketones; methyl 1-methylcyclopentyl ketone, methyl 1-methylcyclohexyl ketone, and other cycloalkyl alkyl ketones; bicyclo [2.2.2]oct-1-yl methyl ketone, decalin-4a-yl methyl ketone, 1-adamantyl methyl ketone, 1-adamantyl ethyl ketone, tricyclo [5.2.1.0^{2,6}]dec-1-yl methyl ketone, and other ketones each having a bridged cyclic group bonding to a carbonyl group at a bridgehead position, and other ketones; 2,2-dimethylpropanal, 1-methylcyclohexane-1-carbaldehyde, 1-adamantanecarbaldehyde, and other aldehydes.

The invented process can smoothly proceed an addition reaction to yield a corresponding alcohol in a high yield, even when the reactant is a carbonyl compound (especially a ketone) having a rigid and bulky hydrocarbon group bonded to a carbonyl group, such as a carbonyl compound having a bridged cyclic group bonding to the carbonyl group at a bridgehead position.

The hydrocarbon groups in R³ in the formula (2) include the groups exemplified as the hydrocarbon groups in R². Preferred hydrocarbon groups are, for example, methyl, ethyl, propyl, isopropyl, butyl, isobutyl, pentyl, 2,2-dimethylpropyl, hexyl, and other C₁-C₆ alkyl groups, and phenyl group, of which methyl group and other C₁-C₄ alkyl groups are particularly preferred.

The ligand L includes, but is not limited to, chlorine, bromine, and other halogen atoms; methoxy, ethoxy, propoxy, isopropoxy, butoxy, isobutoxy, s-butoxy, t-butoxy, pentyloxy, hexyloxy, and other C₁-C₆ alkoxy groups; phenoxy, naphthoxy, and other aryloxy groups; methylthio, ethylthio, isopropylthio, butylthio, and other C₁-C₆ alkylthio groups; phenylthio, naphthylthio, and other arylthio groups; dimethylamino, diethylamino, dipropylamino, diisopropylamino, and other di-C₁-C₄ alkylamino groups; and cyclopentadienyl group. Of these groups, chlorine atom and other halogen atoms, and isopropoxy group, butoxy group, and other C₁-C₆ alkoxy groups are preferred.

In the formula (2), n denotes an integer from 1 to 4. Preferred n is 2 or more (e.g., 2).

Organic titanium compounds of the formula (2) can be prepared by a conventional technique. Such techniques include, for example, a process comprising the steps of the following formulae.

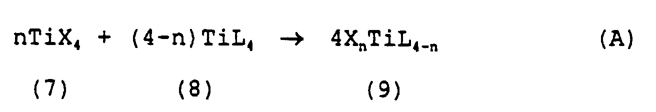

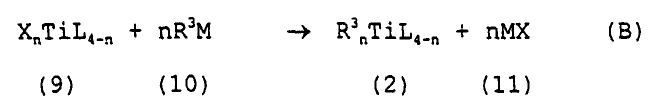

In the formulae, X is a halogen atom (e.g., chlorine or bromine atom), M is Li, MgCl, MgBr or MgI, and L, R³ and n have the same meanings as defined above.

The reaction (A) between a compound of the formula (7) and a compound of the formula (8) in the above process is performed, for example, at a temperature of from about -50°C to 100°C, and preferably from about -10°C to 50°C in the absence of, or in the presence of a solvent. Such solvents include, for example, diethyl ether, dimethoxyethane, tetrahydrofuran, dioxane and other ether solvents; benzene, toluene, xylene, and other aromatic hydrocarbon solvents; hexane, heptane, octane, and other aliphatic hydrocarbon solvents; cyclohexane, and other alicyclic hydrocarbon solvents; and mixtures of these solvents. A compound of the formula (9) obtained through the reaction can be subjected to a subsequent step with or without isolation in a conventional manner.

The reaction (B) between the compound of the formula (9) and a compound of the formula (10) is performed, for example, at a temperature of from about -50°C to 100°C, and preferably from about -10°C to 50°C in a solvent similar to that in the reaction (A). Preferred solvents include ether solvents, or mixtures of an ether solvent with another solvent. The concentration of the ether solvent in the solvent is preferably 10% by weight or more. The amount of the solvent is, for example, about 5 to 50 parts by weight relative to 1 part by weight of the compound of the formula (10). The organic titanium compound of the formula (2) formed through the reaction can be subjected to a reaction according to the invention with or without isolation in a conventional manner.

In the invented alcohol production process 1, a reaction is usually performed in an inert organic solvent. Such organic solvents includes solvents similar to those mentioned in the reaction of the formula (B). The amount of the organic titanium compound of the formula (2) is, for example, about 0.9 to 1.8 moles, and preferably about 1 to 1.4 moles relative to 1 mole of the carbonyl compound of the formula (1).

The reaction can be carried out in any of a batch system, a semi-batch system or a continuous system. The reaction is generally performed by adding either or both the compound of the formula (1) and the compound of the formula (2) dropwise to a reaction system. In a preferred embodiment, the reaction is performed while adding the compound of the formula (1) dropwise to a mixture of the compound of the formula (2) and a solvent. In this embodiment, the carbonyl compound of the formula (1) can be supplied to the reaction system as intact or as a solution in the solvent.

The reaction temperature is, for example, about -50°C to 130°C, preferably about -50°C to 100°C, and more preferably about -10°C to 85°C.

After the completion of the reaction, a reaction product is hydrolyzed to yield a target alcohol. The reaction product can be hydrolyzed for example by adding the reaction mixture to an acidic aqueous solution. Such acidic substances for use herein include, but are not limited to, hydrochloric acid, sulfuric acid, nitric acid, phosphoric acid, and other inorganic acids; ammonium chloride, and other salts of inorganic acids. The proportion of the acidic substance is, for example, about 1 to 10 times by equivalent that of the organic titanium compound of the formula (2) used in the reaction.

After the completion of hydrolysis, the target compound alcohol can be separated and purified by a conventional separation and purification means. Such separation and purification means include extraction, neutralization, concentration, crystallization, filtration, recrystallization, distillation, column chromatography, and combinations of these means. If the alcohol is to be converted into a derivative, the alcohol can be subjected to a subsequent reaction without separation. For example, the alcohol as a concentrated residue can be subjected to the subsequent reaction.

A typical example of the invented alcohol production process 1 is a process which includes the step of reacting a 1-adamantyl ketone derivative having the following formula (1a): 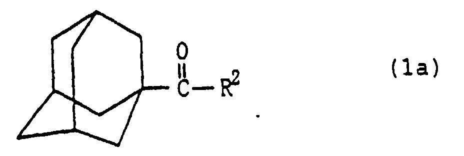 wherein R² has the same meaning as defined above and an adamantane ring may have a substituent,
with the organic titanium compound of the formula (2) to yield an adamantanemethanol derivative of the following formula (3a): 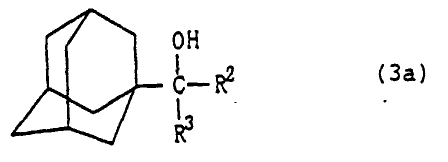 wherein R² and R³ have the same meanings as defined above, and the adamantane ring may have a substituent.

In the formula (4) in the invented alcohol production process 2, hydrocarbon groups in R⁴ include aliphatic hydrocarbon groups, alicyclic hydrocarbon groups, aromatic hydrocarbon groups, and groups comprising a plurality of these groups combined successively. Each of the hydrocarbon groups may have a substituent within a range not deteriorating the reaction.

Such aliphatic hydrocarbon groups include, but are not limited to, methyl, ethyl, propyl, isopropyl, butyl, isobutyl, s-butyl, t-butyl, pentyl, hexyl, octyl, decyl, vinyl, allyl, 2-propynyl, and other C₁-C₁₀ aliphatic hydrocarbon groups (alkyl groups, alkenyl groups, and alkynyl groups). The alicyclic hydrocarbon groups include, for example, cyclopentyl, cyclohexyl, and other alicyclic hydrocarbon groups (cycloalkyl groups and cycloalkenyl groups) having 3 to 8 members. Illustrative aromatic hydrocarbon groups are phenyl, naphthyl, and other C₆-C₁₄ aromatic hydrocarbon groups. The groups comprising a plurality of different hydrocarbon groups combined successively include, for example, benzyl, 2-phenylethyl, and other aralkyl groups having about 7 to 10 carbon atoms.

Among these hydrocarbon groups in R⁴, C₄-C₁₀ alkyl groups and phenyl group are typically preferred. When the substituent R⁴ is any of the preferred groups, the resulting compound of the formula (4) has an extremely high reactivity with the compound of the formula (5). In addition, a corresponding alcohol or phenol (R⁴-OH) can be separated from water or has a high boiling point, and when the compound of the formula (4) is prepared through esterification of 1-adamantanecarboxylic acid and the compound R⁴-OH as described below, a reaction can efficiently proceed while distilling off by-produced water according to a reflux-dehydration method.

Of the compounds of the formula (4), compounds in which R⁴ is a hydrocarbon group can be easily prepared, for example, through a conventional esterification reaction between 1-adamantanecarboxylic acid, where R⁴ is a hydrogen atom, and a corresponding alcohol or phenol using an acid catalyst. Specifically, the material compound of the formula (4) for use in the invented process can be readily obtained from a material that can be easily handled, without the use of halogen agents or the like which by-produce environmentally undesirable substances. The compound of the formula (4) is stable and can be easily handled and stably stored.

The adamantane ring in the compound of the formula (4) may have a substituent. Such substituents include, for example, halogen atoms (e.g., chlorine atom), alkyl groups (e.g., methyl group and other C₁-C₄ alkyl groups), hydroxyl group, alkoxy groups (e.g., methoxy group and other C₁-C₄ alkoxy groups), hydroxymethyl group, substituted or unsubstituted amino groups, carboxyl group, nitro group, acyl groups (e.g., acetyl group and other C₂-C₅ aliphatic acyl groups, benzoyl group and other arylcarbonyl groups), acyloxy groups (e.g., acetoxy group and other C₂-C₅ aliphatic acyloxy groups, benzoyloxy group and other arylcarbonyloxy groups), alkoxycarbonyl groups, and cyano group.

The C₁-C₆ alkyl group in R⁵ in the formula (5) includes, but is not limited to, straight-chain or branched-chain C₁-C₄ alkyl groups such as methyl, ethyl, propyl, isopropyl, butyl, isobutyl, s-butyl, t-butyl, pentyl, and hexyl groups. Preferred R⁵ includes C₁-C₄ alkyl groups. The substituent X includes, for example, chlorine, bromine, and iodine atoms.

A reaction between the compound of the formula (4) and the organic magnesium compound of the formula (5) is usually performed in an inert solvent. Such solvents include, but are not limited to, diethyl ether, dibutyl ether, dimethoxyethane, tetrahydrofuran, and other chain or cyclic ethers; hexane, heptane, octane, and other aliphatic hydrocarbons; benzene, toluene, xylene, and other aromatic hydrocarbons; cyclohexane, and other alicyclic hydrocarbons; and mixtures of these solvents. Preferred solvents include the ethers and mixture solvents of the ether with another solvent. The concentration of the ether in the solvent is preferably 10% by weight or more.

The reaction is performed at temperatures ranging, for example, from about 0°C to 100°C and preferably from about 10°C to 40°C. The proportion of the organic magnesium compound of the formula (5) is about 2 to 4 times by equivalent that of the compound of the formula (4). When the substituent R⁴ in the formula (4) is a hydrogen atom, the proportion of the compound of the formula (5) is about 3 to 5 times by equivalent that of the compound of the formula (4).

The reaction can be performed in a batch system, a semi-batch system, a continuous system, or any other conventional system. When the reaction is performed in a semi-batch system, the compound of the formula (4) may be added (dropwise) to a solution containing the organic magnesium compound of the formula (5) or, alternatively, the organic magnesium compound of the formula (5) may be added (dropwise) to a solution containing the compound of the formula (4).

After the completion of the reaction, a corresponding α,α-dialkyl-1-adamantanemethanol derivative of the formula (6) can be obtained by adding an aqueous solution containing an acid or a salt to the reaction mixture to decompose an adduct of the organic magnesium compound, adjusting acidity or alkalinity of the mixture according to necessity, and subjecting the resulting mixture to a conventional separation and purification means. Such acids include hydrochloric acid, sulfuric acid, and other inorganic acids; acetic acid, and other organic acids, and such salts include ammonium chloride. The conventional separation and purification means include, for example, filtration, concentration, extraction, distillation, crystallization, recrystallization, and column chromatography.

If the adamantanemethanol derivative is to be converted into its derivative, the adamantanemethanol derivative can be subjected to a subsequent reaction without separation. For example, the adamantanemethanol derivative as a concentrated residue can be subjected to the subsequent reaction.

The organic magnesium compound of the formula (5) can be prepared by a conventional technique for use in the preparation of a Grignard reagent. For example, the organic magnesium compound can be prepared by adding, where necessary, a small portion of iodine or another reaction accelerator to a mixture of metallic magnesium, a portion of a compound of the following formula (12), and an organic solvent:

R⁵X (12)

wherein R⁵ and X have the same meanings as defined above, to initiate a reaction, and adding a residual portion of the compound of the formula (12) to the mixture to continue the reaction. Such organic solvents include the solvents for use in the invented process. The proportion of the metallic magnesium is, for example, about 1 to 1.5 moles relative to 1 mole of the compound of the formula (12), and the reaction is performed at temperatures ranging, for example, from about 0°C to 100°C. The prepared organic magnesium compound of the formula (5) can be used in the reaction without isolation.

The invented alcohol production process 1 utilizes a specific organic titanium compound and can smoothly proceed an addition reaction while suppressing side reactions, even when the reactant is a carbonyl compound having a rigid and bulky group such as an adamantyl group. Thus, a corresponding alcohol (e.g., an adamantanemethanol derivative) can be obtained in a high yield. The invented production process 1 can be therefore employed for general purpose as a process for preparing an alcohol through an addition reaction of a carbonyl compound having a quaternary carbon atom at the adjacent position to a carbonyl group.

The invented alcohol production process 2 can produce an adamantanemethanol derivative in a high yield by using, as a reaction material, a compound that can be readily obtained without by-producing environmentally undesirable substances and can be easily handled.

The present invention will now be illustrated in further detail with reference to several inventive examples and a comparative example below, which are not intended limiting the scope of the invention.

### REFERENCE EXAMPLE 1

In a nitrogen atmosphere, 26.74 g (1.100 mol) of metallic magnesium was placed in a flask. A total of 94.94 g (1.00 mol) of methyl bromide was dissolved in 432.72 g of tetrahydrofuran to yield a solution. About 5% by weight of this solution was added to the metallic magnesium in the flask, and the flask was warmed slightly to initiate an initial reaction. The reaction subsided after a while, and the residual portion of the methyl bromide solution in tetrahydrofuran was added dropwise to the reaction mixture while maintaining an internal temperature not exceeding 40°C. After the completion of addition, the resulting mixture was stirred at an internal temperature of 40°C for 2 hours and was then cooled. To the reaction mixture, 392.09 g of tetrahydrofuran was added to yield a 12% by weight methylmagnesium bromide-tetrahydrofuran solution.

### EXAMPLE 1

In a nitrogen atmosphere, 35.82 g (0.126 mol) of tetraisopropoxytitanium (IV) and 40.05 g of benzene were placed into a flask, and 23.90 g (0.126 mol) of titanium(IV) tetrachloride was added dropwise to the mixture at an internal temperature of 10°C to 30°C. After the completion of addition, 40.05 g of tetrahydrofuran was added dropwise to the resulting mixture at an internal temperature of 10°C to 30°C, and the resulting mixture was warmed and was stirred at room temperature for 1 hour to yield a solution.

To the above-prepared solution, 500.00 g (0.503 mol) of a prepared 12% by weight methylmagnesium bromide-tetrahydrofuran solution was added dropwise at an internal temperature of 5°C to 10°C. The methylmagnesium bromide-tetrahydrofuran solution had been prepared from methyl bromide and metallic magnesium. After the completion of addition, the resulting mixture was stirred at an internal temperature of 5°C to 10°C for 1 hour to yield a solution of dimethyldiisopropoxytitanium (IV).

Next, 37.38 g (0.210 mol) of 1-adamantyl methyl ketone was dissolved in 37.38 g of tetrahydrofuran to yield a solution, and the solution was added dropwise to the dimethyldiisopropoxytitanium(IV) solution at an internal temperature of 5°C to 10°C. After the completion of addition, the resulting mixture was warmed and was stirred at a temperature of 25°C to 32°C for 1 hour. The mixture was further heated to a reflux temperature and was stirred at the reflux temperature (67°C to 69°C) for 8 hours.

After cooling, the reaction mixture was added dropwise to a mixture of 204.00 g of benzene and 247.19 g of a 10% by weight sulfuric acid aqueous solution at an internal temperature of 20°C to 35°C. After completion of addition, the resulting mixture was stirred at an internal temperature of 20°C to 35°C for 1 hour, was allowed to stand for 30 minutes and was separated to an aqueous layer and an organic layer.

To the aqueous layer, 204.00 g of benzene was added; the resulting mixture was stirred at room temperature for 30 minutes and was allowed to stand for 15 minutes and was then separated to an aqueous layer and an organic layer. This procedure was repeated again.

The obtained organic layers were mixed and 204.00 g of a saturated sodium chloride aqueous solution and 204.00 g of a 5% by weight potassium carbonate aqueous solution were added to the mixture, the resulting mixture was stirred at room temperature for 30 minutes, was allowed to stand for 15 minutes and was then separated to an aqueous layer and an organic layer.

The organic layer was dried by adding anhydrous sodium sulfate to the organic layer. After drying, the resulting mixture was filtrated, and the filtrate was concentrated under reduced pressures to yield 41.46 g of α,α-dimethyl-1-adamantanemethanol [= 2-(adamant-1-yl)-2-propanol] having a purity of 96.5% by weight. The yield of the α,α-dimethyl-1-adamantanemethanol was 98.0% on the basis of 1-adamantyl methyl ketone.

### [Spectrum data of α,α-dimethyl-1-adamantanemethanol]

GO-MS m/e, CI: 193 (M-1), EI: 179 (M-CH₃), 176 (M-H₂O), 135 (M-[C(OH₃)₂(OH)])

### COMPARATIVE EXAMPLE 1

In a nitrogen atmosphere, 9.03 g (0.024 mol) of cerium(III) chloride heptahydrate was placed in a flask, and the charged material was gradually heated under reduced pressures with stirring to an internal temperature of 135°C. The material was further stirred at this temperature for 2 hours to dehydrate.

The dehydrated material was cooled to room temperature and 21.64 g of tetrahydrofuran was added to the material, and the resulting mixture was stirred at room temperature for 4 hours and was then allowed to stand at room temperature over night.

To the resulting mixture, 23.85 g (0.024 mol) of a 12% by weight methylmagnesium bromide-tetrahydrofuran solution was added dropwise at an internal temperature of 5°C to 10°C. The methylmagnesium bromide-tetrahydrofuran solution had been prepared from methyl bromide and metallic magnesium. After the completion of addition, the mixture was stirred at 5°C to 10°C for 1 hour. Separately, 3.57 g (0.020 mol) of 1-adamantyl methyl ketone was dissolved in 7.21 g of tetrahydrofuran to yield a solution, and the solution was added to the above reaction mixture at an internal temperature of 5°C to 10°C. After the completion of addition, the resulting mixture was stirred at an internal temperature of 5°C to 10°C for 2 hours, was warmed and was stirred at room temperature for 4 hours.

The above reaction mixture was added to 23.54 g of a 5 by weight sulfuric acid aqueous solution at an internal temperature of 20°C to 30°C. After completion of addition, the resulting mixture was stirred at room temperature for 1 hour. The mixture was neutralized with a 5% by weight sodium hydroxide aqueous solution and was then concentrated until the internal temperature reached 100°C.

After cooling to room temperature, 50.00 g of benzene was added to the concentrated residue, and the resulting mixture was stirred for 30 minutes. The mixture was allowed to stand for 15 minutes and was separated to an aqueous layer and an organic layer. The organic layer was washed with 25.00 g of a saturated sodium sulfate aqueous solution and was dried over anhydrous sodium sulfate.

The dried organic layer was filtrated and the filtrate was concentrated under reduced pressures to yield 3.00 g of α,α-dimethyl-1-adamantanemethanol [= 2-(adamant-1-yl)-2-propanol] having a purity of 70.0% by weight. The yield of α,α-dimethyl-1-adamantanemethanol was 54.3% on the basis of 1-adamantyl methyl ketone.

### REFERENCE EXAMPLE 2

In a nitrogen atmosphere, 26.74 g (1.100 mol) of metallic magnesium was placed in a flask. Separately, 64.52 g (1.00 mol) of ethyl chloride was dissolved in 432.72 g of tetrahydrofuran to yield a solution. About 5% by weight of this solution was added to the metallic magnesium in the flask, and the flask was warmed slightly to initiate an initial reaction. The reaction subsided after a while, and the residual portion of the ethyl chloride solution in tetrahydrofuran was added dropwise to the reaction mixture at an internal temperature not exceeding 40°C. After the completion of addition, the resulting mixture was stirred at an internal temperature of 40°C for 2 hours and was then cooled. To the cooled reaction mixture, 218.70 g of tetrahydrofuran was added to yield a 12% by weight ethylmagnesium chloride-tetrahydrofuran solution.

### EXAMPLE 2

In a nitrogen atmosphere, 106.59 g (0.375 mol) of tetraisopropoxytitanium(IV) and 106.59 g of toluene were placed in a flask, and 23.71 g (0.125 mol) of titanium(IV) tetrachloride was added dropwise to the mixture at an internal temperature of -20°C to -10°C. After the completion of addition, 106.59 g of tetrahydrofuran was added dropwise to the resulting mixture at an internal temperature of -20°C to -10°C, and the resulting mixture was stirred for 1 hour to yield a solution.

To the above-prepared solution, 370.13 g (0.50 mol) of a prepared 12% by weight ethylmagnesium chloride-tetrahydrofuran solution was added dropwise at an internal temperature of -20°C to -10°C. The ethylmagnesium chloride-tetrahydrofuran solution had been prepared from ethyl chloride and metallic magnesium. After the completion of addition, the resulting mixture was stirred at an internal temperature of -20C to -10°C for 1 hour to yield a solution of ethyltriisopropoxytitanium(IV).

Next, 89.15 g (0.50 mol) of 1-adamantyl methyl ketone was dissolved in 89.15 g of tetrahydrofuran to yield a solution, and the solution was added dropwise to the ethyltriisopropoxytitanium(IV) solution at an internal temperature of -20°C to -10°C. After the completion of addition, the resulting mixture was stirred at -20°C to -10°C for 8 hours.

The reaction mixture was added dropwise to a mixture of 204.00 g of toluene and a 10% by weight sulfuric acid aqueous solution at an internal temperature of 0°C to 10°C. After the completion of addition, the resulting mixture was stirred at an internal temperature of 0°C to 10°C for 1 hour, was allowed to stand for 30 minutes and was separated to an aqueous layer and an organic layer.

To the aqueous layer, 204.00 g of benzene was added; the resulting mixture was stirred at room temperature for 30 minutes, was allowed to stand for 15 minutes and was then separated to an aqueous layer and an organic layer. This procedure was repeated again.

The obtained organic layers were mixed and 204.00 g of a saturated sodium chloride aqueous solution and 204.00 g of a 5% by weight potassium carbonate aqueous solution were added to the mixture, the resulting mixture was stirred at room temperature for 30 minutes, was allowed to stand for 15 minutes and was then separated to an aqueous layer and an organic layer.

The organic layer was dried by adding anhydrous sodium sulfate to the organic layer. After drying, the resulting mixture was filtrated, and the filtrate was concentrated under reduced pressures to yield 92.80 g of α-ethyl-α-methyl-1-adamantanemethanol [= 2-(adamant-1-yl)-2-butanol] having a purity of 90.0%. The yield of the α-ethyl-α-methyl-1-adamantanemethanol was 80.0% on the basis of 1-adamantyl methyl ketone.

### [Spectrum data of α-ethyl-α-methyl-1-adamantanemethanol]

GC-MS m/e, CI: 207 (M-1), EI: 193 (M-CH₃), 190 (M-H₂O), 179 (M-C₂H₅), 135 (M-[C(CH₃)(C₂H₅)(OH)])

### REFERENCE EXAMPLE 3

A total of 92.58 g (1.00 mol) of butyl chloride was dissolved in 432.72 g of tetrahydrofuran to yield a solution. In a nitrogen atmosphere, 26.74 g (1.100 mol) of metallic magnesium was placed in a flask, and about 5% by weight of the above-prepared solution was added to the metallic magnesium, and the flask was warmed slightly to initiate an initial reaction. The reaction subsided after a while, and the residual portion of the butyl chloride solution in tetrahydrofuran was added dropwise to the reaction mixture at an internal temperature not exceeding 50°C. After the completion of addition, the resulting mixture was stirred at an internal temperature of 50°C for 2 hours and was then cooled. To the reaction mixture, 424.50 g of tetrahydrofuran was added to yield a 12% by weight butylmagnesium chloride-tetrahydrofuran solution.

### EXAMPLE 3

In a nitrogen atmosphere, 106.59 g (0.375 mol) of tetraisopropoxytitanium(IV) and 106.59 g of toluene were placed in a flask, and 23.71 g (0.125 mol) of titanium(IV) tetrachloride was added dropwise to the mixture at an internal temperature of -20°C to -10°C. After the completion of addition, 106.59 g of tetrahydrofuran was added dropwise to the resulting mixture at an internal temperature of -20°C to -10°C, and the mixture was stirred for 1 hour to yield a solution.

To the above-prepared solution, 487.04 g (0.50 mol) of the prepared 12% by weight butylmagnesium chloride-tetrahydrofuran solution was added dropwise at an internal temperature of -20°C to -10°C. The butylmagnesium chloride-tetrahydrofuran solution had been prepared from butyl chloride and metallic magnesium. After the completion of addition, the resulting mixture was stirred at an internal temperature of -20°C to -10°C for 1 hour to yield a solution of butyltriisopropoxytitanium(IV).

Next, 89.15 g (0.50 mol) of 1-adamantyl methyl ketone was dissolved in 89.15 g of tetrahydrofuran to yield a solution, and the solution was added dropwise to the butyltriisopropoxytitanium(IV) solution at an internal temperature of -20°C to -10°C. After the completion of addition, the resulting mixture was stirred at the same temperature, -20°C to -10°C, for 8 hours.

The reaction mixture was added dropwise to a mixture of 204.00 g of toluene and a 10% by weight sulfuric acid aqueous solution at an internal temperature of 0°C to 10°C. After completion of addition, the resulting mixture was stirred at an internal temperature of 0°C to 10°C for 1 hour, was allowed to stand for 30 minutes and was separated to an aqueous layer and an organic layer.

To the aqueous layer, 204.00 g of benzene was added; the resulting mixture was stirred at room temperature for 30 minutes, was allowed to stand for 15 minutes, and was then separated to an aqueous layer and an organic layer. This procedure was repeated again.

The obtained organic layers were mixed and 204.00 g of a saturated sodium chloride aqueous solution and 204.00 g of a 5% by weight potassium carbonate aqueous solution were added to the mixture, the resulting mixture was stirred at room temperature for 30 minutes, was allowed to stand for 15 minutes and was then separated to an aqueous layer and an organic layer.

The organic layer was dried by adding anhydrous sodium sulfate to the organic layer. The dried mixture was filtrated, and the filtrate was concentrated under reduced pressures to yield 91.95 g of α-butyl-α-methyl-1-adamantanemethanol [= 2-(adamant-1-yl)-2-hexanol] having a purity of 90.0%. The yield of the α-butyl-α-methyl-1-adamantanemethanol was 70.0% on the basis of 1-adamantyl methyl ketone.

### [Spectrum data of α-butyl-α-methyl-1-adamantanemethanol]

GC-MS m/e, CI: 235 (M-1), EI: 221 (M-CH₃), 218 (M-H₂O), 179 (M-C₄H₉), 135 (M-[C(CH₃)(C₄H₉)(OH)])

### EXAMPLE 4

A 12% by weight methylmagnesium bromide-tetrahydrofuran solution was prepared from methyl bromide and metallic magnesium. A total of 59.63 g (0.063 mol) of this solution was placed in a flask. Separately, 4.73 g (0.02 mol) of n-butyl 1-adamantanecarboxylate was dissolved in 7.21 g of tetrahydrofuran to yield a solution. This solution was added dropwise to the solution in the flask while maintaining an internal temperature not exceeding 35°C. After completion of addition, the resulting mixture was stirred at room temperature for 1 hour.

The obtained reaction mixture was added dropwise to 32.37 g of a 10% by weight sulfuric acid aqueous solution while maintaining an internal temperature not exceeding 35°C. The resulting mixture was neutralized with a 5% by weight sodium hydroxide aqueous solution and was then separated to an aqueous layer and an organic layer. The aqueous layer was extracted with two portions of 20.00 g of benzene. The extract was added to the organic layer, and the mixture was washed with 20.00 g of a saturated sodium chloride aqueous solution and was dried over anhydrous sodium sulfate. After drying, the mixture was filtrated, and the filtrate was concentrated under reduced pressure to yield α,α-dimethyl-1-adamantanemethanol having a purity of 95.5% in a yield of 88.7% on the basis of n-butyl 1-adamantanecarboxylate.

### EXAMPLE 5

A 13% by weight ethylmagnesium bromide-tetrahydrofuran solution was prepared from ethyl bromide and metallic magnesium. A total of 61.51 g (0.060 mol) of this solution was placed in a flak. Separately, 4.76 g (0.02 mol) of n-butyl 1-adamantanecarboxylate was dissolved in 7.21 g of tetrahydrofuran to yield a solution. This solution was added dropwise to the above-prepared solution in the flask while maintaining an internal temperature not exceeding 35°C. After completion of addition, the resulting mixture was stirred at room temperature for 1 hour.

The obtained reaction mixture was added dropwise to 32.37 g of a 10% by weight sulfuric acid aqueous solution while maintaining an internal temperature not exceeding 35°C. The resulting mixture was neutralized with a 5% by weight sodium hydroxide aqueous solution and was then separated to an aqueous layer and an organic layer. The aqueous layer was extracted with two portions of 22.24 g of benzene. The extract was added to the organic layer, and the mixture was washed with 22.24 g of a saturated sodium chloride aqueous solution and was dried over anhydrous sodium sulfate. After drying, the mixture was filtrated, and the filtrate was concentrated under reduced pressure to yield α,α-diethyl-1-adamantanemethanol having a purity of 48.8% in a yield of 45.5% on the basis of n-butyl 1-adamantanecarboxylate. A reduction product of a reaction intermediate, α-ethyl-1-adamantanemethanol, was by-produced (yield: 52%).

### [Spectrum data of α,α-diethyl-1-adamantanemethanol]

GC-MS m/e: 204, 193, 175, 161, 147, 135, 86, 79, 67, 57, 41

### [Spectrum data of α-ethyl-1-adamantanemethanol]

GC-MS m/e: 194 (M+), 176, 165, 147, 135, 107, 93, 79, 67, 58, 41

## Claims

1. A process for producing an alcohol comprising the step of reacting a carbonyl compound having the following formula (1): 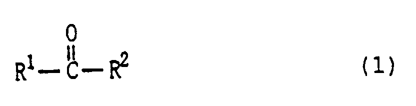 wherein R¹ is a non-aromatic hydrocarbon group having a tertiary carbon atom at a bonding site to a carbonyl group, and R² is a hydrogen atom or a hydrocarbon group,
with an organic titanium compound having the following formula (2):
R³ₙTiL₄₋ₙ (2)
wherein R³ is a hydrocarbon group, L is a ligand, and n denotes an integer of 1 to 4,
to yield an alcohol having the following formula (3): 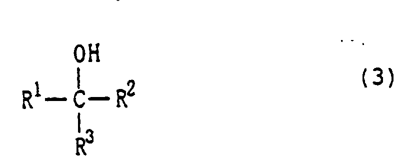 wherein R¹, R² and R³ have the same meanings as defined above.

2. A process for producing an alcohol according to claim 1, wherein R¹ is (a) an aliphatic hydrocarbon group having a tertiary carbon atom at a bonding site to the carbonyl group, (b) an alicyclic hydrocarbon group having a hydrocarbon group bonded to a carbon atom at a bonding site to the carbonyl group, or (c) a bridged cyclic group which is bonded to the carbonyl group at a bridgehead position.

3. A process for producing an alcohol according to claim 1, wherein R² is a hydrogen atom or a C₁-C₁₀ aliphatic hydrocarbon group.

4. A process for producing an alcohol according to claim 1, wherein R³ is a C₁-C₆ alkyl group or a phenyl group.

5. A process for producing an alcohol according to claim 1, wherein L is a group selected from the group consisting of a halogen atom, a C₁-C₆ alkoxy group, an aryloxy group, a C₁-C₆ alkylthio group, an arylthio group, a di-C₁-C₄ alkylamino group, and a cyclopentadienyl group.

6. A process for producing an alcohol according to claim 1, comprising the step of reacting a 1-adamantyl ketone derivative having the following formula (1a): 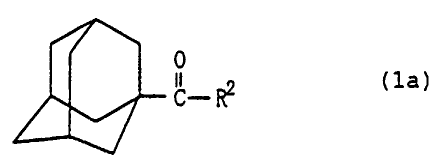 wherein R² is a hydrogen atom or a hydrocarbon group, and an adamantane ring may have a substituent,
with an organic titanium compound having the formula (2) to yield an adamantanemethanol derivative having the following formula (3a): 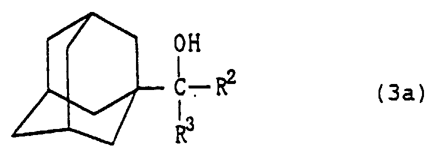 wherein R³ is a hydrocarbon group, R² has the same meaning as defined above, and the adamantane ring may have a substituent.

7. A process for producing an alcohol comprising the step of reacting a 1-adamantanecarboxylic acid derivative having the following formula (4): 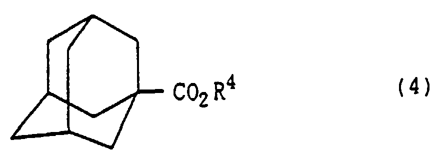 wherein R⁴ is a hydrogen atom or a hydrocarbon group, and an adamantane ring may have a substituent,
with an organic magnesium compound having the following formula (5):
R⁵MgX (5)
wherein R⁵ is a C₁-C₆ alkyl group, and X is a halogen atom, to yield an adamantanemethanol derivative having the following formula (6): 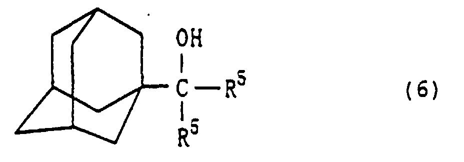 wherein R⁵ has the same meaning as defined above, and the adamantane ring may have a substituent.
